Europäisches Patentamt

European Patent Office

Office européen des brevets

(19)

(11) Numéro de publication : **0 088 022**
**B1**

(12) ## FASCICULE DE BREVET EUROPÉEN

(45) Date de publication du fascicule du brevet :
11.11.87

(51) Int. Cl.⁴ : **C 07 H 15/24, A 61 K 31/70**

(21) Numéro de dépôt : **83400399.8**

(22) Date de dépôt : **25.02.83**

(54) **Agents antitumoraux à efficacité améliorée, leur obtention et procédé pour augmenter l'efficacité des agents antitumoraux.**

(30) Priorité : **26.02.82 FR 8203272**

(43) Date de publication de la demande :
**07.09.83 Bulletin 83/36**

(45) Mention de la délivrance du brevet :
**11.11.87 Bulletin 87/46**

(84) Etats contractants désignés :
**BE CH DE GB IT LI NL**

(56) Documents cités :
**EP-A- 0 004 476**
**EP-A- 0 064 040**
**FR-A- 2 382 450**
**GB-A- 2 017 125**
**US-A- 4 202 967**
**Ariens, "DRUG DESIGN", Vol. X, p. 226-9, 246, 247**
**"Journal of Medicinal Chemistry", Vol. 7, (1964), p. 650-2**
**Chemical Abstracts (1969), 71: 11220p**
**METHODS IN ENZYMOLOGY, Vol. 70, p. 159-165**

(73) Titulaire : **INSTITUT PASTEUR**
**28, rue du Docteur Roux**
**F-75715 Paris Cedex 15 (FR)**

(72) Inventeur : **Relyveld, Edgar Hans**
**3, Place du Général Stefanik**
**F-75016 Paris (FR)**

(74) Mandataire : **Phélip, Bruno et al**
**c/o Cabinet Harlé & Phélip 21, rue de la Rochefoucauld**
**F-75009 Paris (FR)**

EP 0 088 022 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

L'invention concerne le domaine des agents antitumoraux. Dans la présente description, l'expression « agents antitumoraux » est synonyme de l'expression « agents cytostatiques ». La présente invention a pour objet une forme de présentation améliorée de tels agents, qui permet de leur conférer une efficacité augmentée. L'invention concerne également l'obtention des nouvelles formes de présentation des agents en cause et, d'une façon générale, un procédé pour augmenter l'efficacité d'emploi des agents antitumoraux ou cytostatiques.

On sait que l'utilisation pratique des agents antitumoraux actuels soulève de nombreuses difficultés. A des doses suffisantes pour être efficaces, de tels agents sont extrêmement toxiques. Leur emploi se justifie cependant en raison de la gravité des affections contre lesquelles il faut lutter. Par ailleurs, les conditions mêmes d'un traitement clinique sont loin d'être optimales car, dans de nombreux cas, les agents actifs s'éliminent très vite de l'organisme, de sorte qu'il est nécessaire de les appliquer plus fréquemment, ce qui augmente encore les risques de toxicité et, en tout cas, rend le traitement très inconfortable pour le malade.

Dans des travaux récents, diverses tentatives ont été faites pour remédier à ces inconvénients, en particulier en fixant l'agent antitumoral, par exemple la daunomycine (ou daunorubicine) sur divers supports solubles ou insolubles. On peut citer à cet égard l'article de A. Bernstein et al., J. Natl. Cancer Inst. 1978, 60, 379-384. Les auteurs proposent de fixer la daunomycine sur du dextran pour augmenter l'efficacité antitumorale. En vue de la fixation, le dextran, préalablement oxydé, est mis en présence de la daunomycine et l'ensemble est ensuite réduit par le borohydrure de sodium. Lors de l'administration, les auteurs constatent un certain nombre d'effets secondaires indésirables, en raison de la présence du dextran comme support.

A titre de référence bibliographique, on peut citer également l'article de Hurwitz E., Wilchek M. and Pitha J. Soluble Macromolecules as Carriers for Daunorubicin. J. Appl. Biochem. 2,25-35 (1980). Selon ces auteurs, la daunorubicine est fixée par son groupe cétonique sur des hydrazides macromoléculaires solubles, et cela d'une manière réversible. La fixation a lieu, par conséquent, par des liaisons sulfures. Les difficultés rencontrées sont que le produit ne possède plus ses propriétés cytotoxiques ou encore que le conjugué devient progressivement insoluble, ce qui le rend impropre à l'application.

L'article de Sjur Olsnes « Directing toxins to cancer cells » dans Nature Vol. 290 — 12 mars 1981, décrit bien les problèmes liés à l'administration des agents antitumoraux dont la toxicité est très élevée. Il évoque un certain nombre de moyens pour essayer d'améliorer les conditions d'application, et en particulier pour permettre une action plus directe de l'agent actif sur la cellule cancéreuse en améliorant sa sélectivité. Il est proposé, par exemple, de fixer l'agent antitumoral sur une toxine capable d'agir directement sur la cellule.

On peut citer également l'article de Jung-Yaw Lin, Jiann-Shyong Li and Ta-Cheng Tung « Lectin Derivatives of Methotrexate and Chlorambucil as Chemotherapeutic Agents » JNCI Vol. 66, n° 3, mars 1981. Les auteurs proposent de fixer les agents sur une lectine, par liaison covalente, de manière à préparer un produit conjugué ayant une efficacité supérieure à l'agent actif utilisé séparément.

D'une manière générale, on connaît divers procédés, surtout ces dernières années, ayant pour but de transporter des médicaments, de retarder leur élimination, de diminuer leur toxicité, d'augmenter leur activité, notamment. Ces études ont entre autres fait l'objet d'un ouvrage récent : Drug Carriers in Biology and Medicine, G. Gregoriadis ed. Academic Press 1979, qui résume les résultats obtenus avec des macromolécules naturelles, des cellules et des composants synthétiques (tels que les liposomes).

D'autres voies ont fait l'objet de publications récentes : la chimioembolisation artérielle avec des microcapsules contenant un anticancéreux (T. Kato et al. JAMA 1981, 245, 1123-1127), les anticorps porteurs de toxines ou d'agents anticancéreux (H. E. Blythman et al., NATURE 1981, 290, 145-146 ; S. Olsnes, NATURE 1981, 290, 84 ; E. Hurwitz et al. Europ. J. Cancer 1978, 14, 1213-1220), l'utilisation de pompes implantables (JAMA 1981, 246, 925-926).

Les problèmes posés par l'administration des agents antitumoraux ou cytostatiques sont rendus particulièrement difficiles par la nature de la maladie et la toxicité très élevée du produit actif. L'invention a pour objet un moyen pour améliorer l'efficacité d'emploi d'agents antitumoraux, de manière à permettre une libération progressive de ceux-ci dans l'organisme, ce qui améliore nettement leur efficacité et le confort du malade.

On connaît déjà le concept général de la transformation de certains agents thérapeutiquement actifs pour leur conférer une présentation sous forme d'un polymère insoluble biodégradable, l'agent actif étant libéré progressivement après administration à l'organisme (voir « Drug Design » volume X, pages 226-9 et 246, 247). Ce concept a déjà été appliqué, dans le domaine des traitements anticancéreux, à un agent actif particulier, la p-sarcolysine (voir Journal of Medicinal Chemistry, volume 7 (1964), pages 650-652).

Toutefois il n'a pas été proposé jusqu'à présent des produits antitumoraux à efficacité améliorée obtenus après réaction avec le glutaraldéhyde.

A titre de documents illustrant la technique antérieure, on peut encore citer le brevet français publié sous le n° 2 382 450 et la demande de brevet européen publiée sous le n° 0 004 467.

Le brevet FR-2 382 450 a pour objet une famille de nouveaux dérivés de la pyridine, susceptibles d'être utilisés comme agents bifonctionnels, notamment pour se substituer au glutaraldéhyde, lequel est un agent bien connu à cet effet. Il concerne une catégorie particulière d'agents bifonctionnels et ne contient aucun enseignement pour l'obtention de produits antitumoraux. En outre, sa description critique l'efficacité du glutaraldéhyde comme agent de pontage. L'homme du métier aurait donc pu s'attendre à rencontrer certaines difficultés lors de l'utilisation d'un tel agent.

Selon la présente invention, le glutaraldéhyde convient parfaitement pour la préparation des produits antitumoraux. La demande de brevet européen publiée sous le n° 0 004 467, au nom de l'Université de Californie décrit une nouvelle forme de présentation d'antitumoraux, tels que la daunorubicine et l'adriamycine, et prévoit de coupler deux molécules du produit de base pour réaliser un dimère. Celui-ci peut être incorporé dans un liposome, ou présenté en solution aqueuse, ce qui implique nécessairement que ces nouveaux dérivés dimères sont solubles dans les milieux aqueux. Il est bien établi dans le brevet de l'Université de Californie, que les nouveaux composés sont des bis-anthracyclines dont la caractéristique essentielle est d'être formés de deux unités d'anthracyclines reliées entre elles par un pontage.

Une différence fondamentale des agents antitumoraux selon l'invention vis-à-vis des composés faisant l'objet du brevet de l'Université de Californie, est que lesdits agents sont des produits insolubles en milieu aqueux. La mise en suspension dans un tel milieu permet de libérer progressivement l'agent actif tout en conservant son activité antitumorale. Les produits polymères selon l'invention sont insolubles en milieu aqueux. Cette propriété est particulièrement avantageuse en thérapeutique humaine. L'agent antitumoral étant insoluble en milieu aqueux se dépolymérise progressivement lorsqu'il est en contact avec les fluides physiologiques, ce qui permet au produit actif de se remettre en solution pour exercer sa pleine efficacité.

Le brevet EP-0 004 467 ne divulgue aucunement les caractéristiques et les propriétés de tels produits antitumoraux pas plus que les moyens de les obtenir.

Le brevet US-A 4 202 967 décrit un procédé pour obtenir de nouveaux dérivés de daunomycine et d'adriamycine. Ceux-ci sont des composés bien définis qui résultent de la réaction des agents précités avec du glutaraldéhyde et du cyanoborohydrure de sodium. Par exemple, les composés sont obtenus en faisant réagir des quantités équimolaires de glutaraldéhyde et de l'agent antitumoral (daunorubicine ou adriamycine) avec addition consécutive de cyanoborohydrure de sodium. Il est précisé également que les composés ainsi préparés sont solubles dans l'eau. Ce document antérieur n'enseigne donc pas la préparation d'un composé insoluble dans l'eau, susceptible de procurer un effet retard grâce à une libération progressive de l'agent actif. Les conditions réactionnelles mises en œuvre dans le brevet US-A 4 202 967 impliquent l'utilisation du cyanoborohydrure de sodium ainsi que des quantités équimolaires de glutaraldéhyde et d'agent antitumoral. Ce brevet antérieur n'enseigne pas des conditions réactionnelles, en particulier une concentration en glutaraldéhyde qui, en l'absence de cyanoborohydrure de sodium, permet d'obtenir un produit final insoluble.

L'invention concerne des agents antitumoraux à efficacité améliorée, caractérisés en ce qu'ils consistent en le produit de la réaction avec du glutaraldéhyde, d'au moins un agent antitumoral, porteur de groupes capables de réagir avec le glutaraldéhyde, en choisissant les conditions de réaction entre l'agent antitumoral à traiter et le glutaraldéhyde, et spécialement la quantité de glutaraldéhyde, en utilisant une quantité minimale de celui-ci, supérieure à celle qui laisse dans le milieu réactionnel des quantités résiduelles non réagies d'agent antitumoral, sans dépasser les quantités excessives de glutaraldéhyde susceptibles de conduire à un produit soluble en milieu aqueux par saturation des groupements réactifs de l'agent antitumoral, aboutissant à un produit insoluble en milieu aqueux, lequel produit, après élimination du glutaraldéhyde éventuel en excès, permet, mis en suspension dans un milieu aqueux, de libérer progressivement l'agent actif sous une forme soluble en conservant son activité antitumorale.

L'invention a également pour objet un procédé pour l'obtention des agents antitumoraux ci-dessus mentionnés dans lequel on soumet l'agent actif à une réaction de polymérisation, caractérisé en ce qu'on met un agent antitumoral, porteur de groupes capables de réagir avec le glutaraldéhyde, en contact avec du glutaraldéhyde, en choisissant les conditions de réaction entre l'agent antitumoral à traiter et le glutaraldéhyde, et spécialement la quantité de glutaraldéhyde, en utilisant une quantité minimale de celui-ci, supérieure à celle qui laisse dans le milieu réactionnel des quantités résiduelles non réagies d'agent antitumoral, sans dépasser les quantités excessives de glutaraldéhyde susceptibles de conduire à un produit soluble en milieu aqueux par saturation des groupements réactifs de l'agent antitumoral, et en ce qu'on récupère un produit insoluble en milieu aqueux, lequel produit, après élimination du glutaraldéhyde éventuel en excès, permet, mis en suspension dans un milieu aqueux, de libérer progressivement l'agent actif sous une forme soluble en conservant son activité antitumorale.

Dans tous les aspects de l'invention mentionnés ci-dessus, il est essentiel que le glutaraldéhyde soit capable de réagir avec l'agent antitumoral à traiter.

L'invention est applicable aux agents antitumoraux les plus divers dès qu'ils sont porteurs de groupes capables de réagir avec le glutaraldéhyde.

A titre d'exemples d'agents antitumoraux particuliers, on peut citer la daunorubicine, également dénommée daunomycine (voir Merck Index n° 2815 année 1976) ainsi que des dérivés similaires, tels que la doxorubicine ou adriamycine (Merck Index n° 3428 année 1976). On peut également citer les produits méthotrexate (Merck Index n° 5858, année 1976) et Mitomycine I (Merck Index n° 6060, année 1976).

Selon un mode préféré de réalisation de l'invention, l'agent antitumoral à traiter est mis en contact avec le glutaraldéhyde dans des conditions bien définies. Ainsi qu'on l'a mentionné précédemment, l'agent antitumoral doit être du type capable de réagir avec le glutaraldéhyde. Selon l'art antérieur, et notamment dans les articles mentionnés au début de la présente description, la fixation des agents antitumoraux sur certains supports se faisait par des réactions organiques ou d'affinité. Selon la présente invention, la réaction avec le glutaraldéhyde conduit à une forme de l'agent tumoral d'origine, laquelle, contrairement à l'agent de départ, est insoluble dans les milieux aqueux, en particulier les fluides biologiques ou les solutés physiologiques.

Les conditions de mise en présence de l'agent antitumoral et du glutaraldéhyde doivent non seulement conduire à un produit insoluble, mais également être telles que ledit produit étant ensuite, en l'absence de glutaraldéhyde, mis en suspension dans un milieu aqueux, soit à même de libérer l'agent actif sous une forme soluble, en conservant son activité antitumorale. On doit donc choisir les conditions de réaction et en particulier la quantité de glutaraldéhyde mis en œuvre, de façon à satisfaire aux exigences susmentionnées. Si l'on utilise un excès de glutaraldéhyde, on aboutit à une saturation des groupements réactifs de l'agent antitumoral sur lesquels se fixent les groupes de glutaraldéhyde et l'on obtient en définitive un produit soluble dans les milieux aqueux. Au contraire, si l'on utilise une quantité trop faible de glutaraldéhyde, celui-ci n'est pas capable de fixer tous les groupes réactifs de toutes les molécules de l'agent antitumoral, de sorte qu'il subsiste des molécules libres de cet agent qui n'ont pas réagi. L'homme de l'art comprendra que le juste milieu à adopter pour choisir la quantité de glutaraldéhyde à mettre en œuvre dépendra de la nature de l'agent antitumoral, en particulier du nombre de groupes réactifs qu'il possède. Des essais préalables permettent de déterminer les quantités optimales de glutaraldéhyde, celles-ci devant se situer entre deux extrêmes, d'une part celles en excès, qui conduisent à un produit soluble en milieu aqueux et, d'autre part, celles en défaut, qui laissent dans le milieu réactionnel des quantités résiduelles non réagies d'agent antitumoral.

Il va sans dire également que la durée de réaction peut jouer un certain rôle. La réaction doit être poursuivie entre l'agent antitumoral et le glutaraldéhyde jusqu'à ce que les exigences ci-dessus soient satisfaites.

Dans les exemples qui suivent, on a illustré la réaction entre le glutaraldéhyde et la daunorubicine. On a essayé trois concentrations en glutaraldéhyde, respectivement de 0,027 % en poids, de 0,27 % et de 2,7 % par rapport au milieu réactionnel qui contenait 1 mg/ml de daunorubicine. Avec des temps de contact à la température ambiante de 15 min et de 30 min, les conditions de réaction qui convenaient aux besoins de l'invention impliquaient l'utilisation de concentrations en glutaraldéhyde de 0,27 % environ. Avec une telle concentration, en effet, la quantité de produit insoluble atteignait près de 100 %. En revanche, à la concentration en glutaraldéhyde de 0,027 % la quantité de produit insoluble était insuffisante et il demeurait de la daunorubicine libre non réagie. De même à des concentrations de 2,7 % en glutaraldéhyde, la quantité de produit restant soluble dans le milieu aqueux était devenue importante, ce qui n'était pas satisfaisant.

Les indications qui précèdent sont données à l'homme de l'art qui pourra, dans chaque cas particulier, et par des essais préalables, définir les conditions optimales pour la réaction selon l'invention entre l'agent antitumoral et le glutaraldéhyde.

Une autre caractéristique très importante de l'invention est que le produit obtenu est capable, après élimination du glutaraldéhyde éventuel en excès, et une fois mis en suspension dans un milieu aqueux, de libérer progressivement l'agent actif sous forme soluble en conservant l'activité antitumorale de celui-ci. Ainsi, l'agent cytostatique actif se libère progressivement lorsque le produit insoluble est mis en soluté physiologique, comme cela est le cas en application thérapeutique. Les conditions réactionnelles mentionnées ci-dessus permettent de ne pas inhiber l'effet thérapeutique et, au contraire, d'augmenter l'efficacité d'emploi des agents antitumoraux.

La fixation du glutaraldéhyde sur l'agent antitumoral peut s'effectuer directement ou indirectement. Le moyen le plus simple consiste à mettre en présence, en particulier à température ambiante, l'agent antitumoral à traiter avec la quantité convenable de glutaraldéhyde et à poursuivre la réaction jusqu'à ce que le produit insoluble soit formé et que l'on ne décèle plus, ou sensiblement plus, d'agent antitumoral non réagi.

En variante, on peut également effectuer la réaction de l'agent actif et du glutaraldéhyde en présence d'un support.

Pour les besoins de l'invention, on utilise de préférence, à titre de support, des cellules et plus particulièrement des lymphocytes. Cette technique de fixation est notamment décrite dans la demande de brevet français FR-A-2 478 470 du 24 mars 1980 au nom du présent demandeur ayant pour titre « Préparations cellulaires applicables en thérapeutique humaine, procédé d'obtention et applications ». On peut se reporter également au brevet suisse 650 932 du 15 juillet 1980, correspondant à la demande de brevet français précitée. D'autres détails sur cette technique se trouvent dans la publication de E. H. Relyveld et S. Ben-Efraim — Preparation of highly immunogenic protein conjugates by direct coupling to glutaraldehyde-treated cells : comparison with commonly used preparations. J. Immunol. Methods 1981, 40, 209-217. Ces préparations cellulaires sont constituées de cellules sur lesquelles sont fixées, à leur surface, des substances ayant une activité pharmacologique. A titre de cellules, on fait appel de préférence à des lymphocytes normaux ou leucémiques. La fixation est effectuée en présence d'un agent de pontage, lequel est de préférence le glutaraldéhyde.

Selon cette variante de mise en œuvre de l'invention, le produit insoluble se présente sous la forme d'une préparation cellulaire, de préférence lymphocytaire, portant l'agent antitumoral sous forme couplée. De la même manière que dans le cas d'une fixation directe du glutaraldéhyde sur l'agent antitumoral, le produit insoluble obtenu conformément à ladite variante, peut, s'il est mis en suspension dans un milieu aqueux en absence de glutaraldéhyde, comme cela peut être le cas, par exemple dans l'organisme, libérer progressivement l'agent antitumoral en conservant à celui-ci son activité cytostatique.

Les expériences rapportées ci-après concernent la daunorubicine. Les résultats obtenus suivant les protocoles classiques pour mesurer l'effet thérapeutique de la daunorubicine ont montré que les produits de l'invention à base de daunorubicine sont plus toxiques que la daunorubicine libre. Cependant, ils permettent d'obtenir avec des quantités moindres, par exemple de l'ordre de la moitié des quantités correspondantes de daunorubicine libre, des résultats thérapeutiques sensiblement équivalents. L'effet thérapeutique de la daunorubicine polymérisée est également meilleur si on le compare à celui de la même quantité du médicament injecté par doses successives, comme cela est le cas dans les traitements habituels, ou en une seule fois. L'effet « retard » procuré par les produits de l'invention permet donc d'améliorer très sensiblement les conditions du traitement et en particulier le confort du malade.

Les produits de l'invention ont les mêmes applications que les agents antitumoraux dont ils dérivent. Ils se prêtent à toutes les formes de mise en œuvre de tels agents tumoraux, par exemple l'injection du dérivé cytotoxique in situ, en particulier dans des tumeurs solides ou encore dans les traitements mentionnés au début de la présente description impliquant la chimioembolisation ou des pompes implantables.

Dans les exemples qui suivent, on a illustré l'invention avec la daunorubicine (ou son chlorhydrate) comme agent antitumoral.

## Exemple 1

### Obtention d'un produit polymère par action de glutaraldéhyde sur la daunorubicine

On met en contact à la température ambiante 1 mg/ml de daunorubicine (chlorhydrate) en solution aqueuse avec du glutaraldéhyde à diverses concentrations, à savoir 0,027 %, 0,27 % et 2,7 % en poids respectivement, pendant 15 min ou 30 min. Les résultats sont rassemblés dans le Tableau 1.

Les résultats du Tableau 1 montrent que la concentration de 0,27 % en poids de glutaraldéhyde est, parmi les trois concentrations essayées la seule qui convienne selon l'invention pour obtenir pratiquement 100 % de produit polymère insoluble.

## Exemple 2

### Fixation de la daunorubicine sur cellules

On a effectué le couplage de la daunorubicine (chlorhydrate) aux cellules leucémiques L 1210 de la souris C57 BL/6 XDBA/2 $F_1$. Les conditions de réaction et les résultats obtenus sont rassemblés au Tableau 2. On constate que la daunorubicine se fixe sur les cellules et se polymérise.

Les résultats du Tableau 2 montrent encore l'importance de la concentration en glutaraldéhyde. Avec $2 \times 10^7$/ml de cellules et 1 mg/ml de daunorubicine, on n'obtient pas de polymère fixé à la concentration de 0,027 %, quelle que soit la durée de réaction, alors que la concentration de 0,27 % fournit un polymère. De même la concentration de 0,108 % en glutaraldéhyde pour $8 \times 10^7$ cellules/ml et 1 mg/ml de daunorubicine ne fournit pas de produit précipité, si l'action du glutaraldéhyde est inhibée par une dilution rapide après la période de contact.

Dans la description qui suit, on a rassemblé dans les tableaux 3 à 7 ci-après, les résultats obtenus dans des essais pharmacologiques permettant de mesurer de façon comparée la toxicité et l'activité antitumorale de la daunorubucine libre, de la daunorobucine polymère (produit de l'invention obtenu par traitement direct de la daunorubicine au glutaraldéhyde) et de la daunorubicine greffée sous forme polymère (produit de l'invention obtenu par fixation préalable de la daunorubicine sur les cellules L 1210 et polymérisation). Les conditions des essais sont indiquées dans les Tableaux, avec les résultats et observations.

Les produits essayés sont injectés par voie i. p. à des souris.

Pour les essais rapportés dans le Tableau 4, on a préparé la daunorubicine polymère conformément à l'exemple 1 à partir d'une quantité de 1 mg/ml de daunorubicine et d'une concentration en glutaraldéhyde de 0,27 % en poids. Les conditions de traitement étaient les suivantes :
— daunorubicine libre : 1 injection de 1 mg/kg aux jours J 1.2.3.4. une fois par jour
— daunorubicine polymère : 1 injection de 1 mg/kg aux jours J 1.2.3.4., une fois par jour
— daunorubicine polymère : 1 injection de 4 mg/kg à J 1. Au jour J 0 les souris étaient inoculées par $10^5$ de cellules leucémiques L 1210.

Il en est de même pour le tableau 5 où les traitements ont lieu comme suit :

— daunorubicine libre : 1 mg/kg J 1.2.3.4.
— daunorubicine polymère :
1 mg/kg J 1.4.7.
0,25 mg/kg J 1.3.5.7.9.
0,25 mg/kg J 1.2.3.4.

Dans le tableau 6, après injection à J 0 de $10^5$ cellules leucémiques, on a comparé les résultats obtenus par les traitements suivants :
— daunorubicine libre 1 mg/kg J 1.2.3.4.
— daunorubicine polymère 0,5 mg/kg J 1.2.3.4.
— daunorubicine polymère 4 mg/J 1.
— daunorubicine polymère greffée 0,5 mg/kg J 1.2.3.4.

Dans le tableau 7, on a comparé les résultats des traitements par chimiothérapie dans les cas suivants :
— témoins épreuve injection de $10^5$ cellules leucémiques à J 0
— témoins daunorubicine libre 1 mg/kg J 1.2.3.4.
daunorubicine libre 4 mg/kg J 1
daunorubicine polymère 4 mg/kg J 1.

Les résultats des tableaux 3 à 7 montrent que, d'une manière générale, les agents antitumoraux selon l'invention consistant en daunorubicine polymère, greffée ou non, sont plus toxiques que la daunorubicine libre. Mais dans le traitement chimiothérapeutique, on obtient des résultats équivalents à ceux de la daunorubicine libre en utilisant une quantité quatre fois moindre de daunorubicine polymère, greffée ou non ou en injectant en une seule fois une quantité égale à la daunorubicine libre injectée successivement (4 mg/kg au jour 1, au lieu de 1 mg/kg aux jours 1.2.3.4.). Le traitement à la daunorubicine polymère conduit à un nombre de souris survivantes au moins égale à celui obtenu avec de la daunorubicine libre. L'efficacité d'emploi de la daunorubicine est donc nettement améliorée par l'invention.

Il va sans dire que l'invention n'est pas limitée aux exemples qui précèdent, lesquels sont relatifs à la daunorubicine (chlorhydrate). L'invention est en effet applicable d'une manière générale à tous les agents antitumoraux porteurs de groupes capables de réagir avec le glutaraldéhyde. De même le greffage de produit polymère n'est pas limité aux lymphocytes, normaux ou leucémiques. On peut, en effet utiliser diverses préparations cellulaires conformément à l'enseignement de la demande de brevet français FR-A-2 478 470 précitée.

On a encore effectué d'autres études sur l'activité retard de la daunorubicine traitée selon l'invention (ou daunorubicine polymère). Les essais ont été réalisés comme ci-dessus sur la leucémie expérimentale L 1210 de la souris.

Le protocole ci-après a été appliqué :

Essai 1 - témoin 1 : greffe de $1 \times 10^5$ cellules L 1210 par voie i. p. Jour 5 ($J_5$)
Essai 2 - témoin 2 : greffe L 1210, $J_5$ ; Daunorubicine monomère 2 mg/kg, $J_6$
Essai 3 - témoin 3 : greffe L 1210, $J_5$ ; Daunorubicine polymère 2 mg/kg, $J_6$
Essai 4 - Daunorubicine monomère 2 mg/kg, $J_3$ ; greffe L 1210, $J_5$
Essai 5 - Daunorubicine polymère 2 mg/kg, $J_3$ ; greffe L 1210, $J_5$

Les résultats obtenus après les épreuves ci-dessus ont été rassemblés dans le tableau 8 qui suit. Les données du tableau 8 montrent que les effets thérapeutiques de la daunorubicine monomère (DM) et le polymère (DP), administrées après la greffe tumorale, sont sensiblement identiques (essais 2 et 3). L'effet retard de la daunorubicine polymère est très significatif ; l'activité thérapeutique est élevée dans des conditions où le produit monomère est sans effet (comparer essais 4 et 5). L'activité thérapeutique est la même dans les essais 3 et 5.

Tableau 1

| Temps de contact avec glutaral-déhyde | concen-tration glutaral-déhyde % | Quantité polymé-risée % | % daunorubicine libérée | | | | | |
|---|---|---|---|---|---|---|---|---|
| | | | 2 h à tem-pérature ambiante | + 2 h 37 °C | + 2 h 37 °C | + 2 h 37 °C | + 2 h 37 °C | + 1 nuit à + 4 °C |
| | 0.027 | 68.75 | 10.9 | 6.5 | 4.9 | 3.8 | 3 | 8.1 |
| 15 min | 0.27 | 97.4 | 7.2 | 7.3 | 8.6 | 4.9 | 4.1 | 11.3 |
| | 2.7 | 57.4 | 6.5 | 7.8 | 5.2 | 5.2 | 3.9 | 6.9 |
| | 0.027 | 80 | 9.4 | 7 | 5.1 | 4.2 | 3.75 | 10.25 |
| 30 min | 0.27 | 97.4 | 4.6 | 5 | 5 | 4.6 | 4.6 | 18.5 |
| | 2.7 | 73.5 | 5.6 | 4.5 | 4.6 | 5.6 | 3.9 | 10.6 |

6

**0 088 022**

Tableau 2

| Concen-tration glutaral-déhyde | Concen-tration cellules | Concen-tration daunoru-bicine | Temps de contact préalable avec cellules | Temps de contact total | Aspect des cellules au microscope | % daunoru-bicine fixée sur les cellules et polymérisée |
|---|---|---|---|---|---|---|
| 0,27 % | $2 \times 10^7$/ml | 1 mg/ml | 0 | 15 min | Cellules incolores présence de dauno-rubicine polymérisée | |
| 0,027 % | $2 \times 10^7$/ml | 1 mg/ml | 0 | 15 min | cellules oranges + pas de polymère | |
| | | | 5 min | 15 min | cellules oranges + + pas de polymère | |
| | | | 10 min | 15 min | cellules oranges + + + pas de polymère | |
| 0,027 % | $2 \times 10^7$/ml | 0,5 mg/ml | 0 | 15 min | quelques cellules colorées | |
| | | 0,250 mg/ml | 0 | 15 min | | |
| | | 0,125 mg/ml | 0 | 15 min | | |
| 0,0027 % | $2 \times 10^7$/ml | 1 mg/ml | 0 | 15 min | cellules non colorées | |
| | | | 5 min | 15 min | | |
| | | | 15 min | 15 min | | |
| 0,027 % | $2 \times 10^7$/ml | 1 mg/ml | 5 min | 15 min | — | 59,4 |
| 0,054 % | $2 \times 10^7$/ml | 1 mg/ml | 5 min | 15 min | — | 81,8 |
| 0,108 % | $2 \times 10^7$/ml | 1 mg/ml | 5 min | 15 min | | 84,8 |
| 0,108 % | $2 \times 10^7$/ml | 1 mg/ml | 5 min | 15 min | | |
| 0,027 % | $8 \times 10^7$/ml | 1 mg/ml | 0 | 15 min | quelques cellules colorées | |
| | | | 5 min | 15 min | quelques cellules colorées | |
| | | | 10 min | 15 min | quelques cellules colorées | 37,4 |
| 0,054 % | $8 \times 10^7$/ml | 1 mg/ml | 0 | 15 min | quelques cellules colorées | |
| | | | 5 min | 15 min | cellules bien colorées | 68,8 |
| | | | 10 min | 15 min | cellules faiblement colorées | 47,5 |
| 0,108 % | $8 \times 10^7$/ml | 1 mg/ml | 0 | 15 min | quelques cellules colorées | |
| | | | 5 min | 15 min | cellules bien colorées | 87 |
| | | | 10 min | 15 min | cellules bien colorées | 80,3 |
| 0.108 % | $8 \times 10^7$/ml | 1 mg/ml | 5 min | 15 min | | 84,8 |
| 0.216 % | $8 \times 10^7$/ml | 1 mg/ml | 5 min | 15 min | | 80,5 |
| 0.432 % | $8 \times 10^7$/ml | 1 mg/ml | 5 min | 15 min | | 86,5 |
| 0.108 % | $8 \times 10^7$/ml | 1 mg/ml | 5 min | 15 min | | 64,4 |

0 088 022

## Tableau 3

### Toxicité de la Daunorubicine libre, Daunorubicine polymère, Daunorubicine greffée polymère

| | Quantités injectées (mg/kg) | Injections et observations | | | | Observations | | | | | | | | MTS * |
|---|---|---|---|---|---|---|---|---|---|---|---|---|---|---|
| | | J.1 | J.2 | J.3 | J.4 | J.5 | J.6 | J.7 | J.8 | J.10 | J.12 | J.13 | J.14 | |
| **Daunorubicine libre** | 1 | 1re inj. | 2e inj. RAS | 3e inj. RAS | 4e inj. RAS | — | — | — | — | — | — | — | — | toutes survivantes |
| | 3 | '' | 2e inj. RAS | 3e inj. 1 = maigre 2 = diarrhée reste RAS | 4e inj. 1 = maigre 2 = diarrhee reste RAS | — | — | 1 M | — | 2 M | 2 M | 4 M | 1 M | 10.7 |
| | 9 | '' | 2e inj. (malades diarrhee ±) | 3e inj. plus de diarrhée maigres | 3e inj. diarrhée finie maigres | — | 2 M | 3 M | 5 M | — | — | — | — | 6.3 |
| **Daunorubicine greffée polymère** | 1 | 1re inj. | 2e inj. RAS | 3e inj. 1 = diarrhée reste RAS | 4e inj. RAS | — | — | — | — | — | | | | Toutes survivantes |
| | 3 | '' | 2e inj. RAS | 3e inj. (maigres diarrhée) | 4e inj. malades-maigres diarrhée | — | — | 2 M | 8 M | — | | | | 6.8 |
| | 9 | '' | 2e inj. (malades diarrhée + +) | pas de 3e inj. diarrhée+ + + + très malades | (9 souris) 3e inj. 1 M | — | 8 M | — | — | — | | | | 4.8 |
| **Daunorubicine polymère** | 1 | 1re inj. | 2e inj. RAS | 3e inj. RAS | 4e inj. 1 = maigre diarrhée | — | 1 M | — | — | — | — | — | — | 9 survivantes |
| | 3 | '' | 2e inj. RAS. | 3e inj. 2 = maigres diarrhée | 4e inj. 1 M malades maigres | — | 1 M | 4 M | 1 M | — | — | — | — | 6.75 (1 survivante) |
| | 9 | '' | 2e inj. (malades diarrhée ±) | pas 3e inj. diarrhée+ + + + très malades | 3e inj. maigres diarrhée | 1 M | 3 M | 4 M | 1 M | — | — | — | — | 5.55 (1 survivante) |

\* temps moyen de survie

\*\* M = mort

Tableau 4

| Préparations | Souris survivantes après l'épreuve | | | | | | | | | | | | | | | MTS | ILS *<br>% |
| | 8e j | 9e j | 10e j | 12e j | 13e j | 14e j | 15e j | 17e j | 19e j | 20e j | 21e j | 24e j | 26e j | 27e j | > 60<br>(J 3e) | | |
|---|---|---|---|---|---|---|---|---|---|---|---|---|---|---|---|---|---|
| Témoins | 10/10 | 10/10 | 10/10 | 7/10 | 6/10 | 3/10 | 1/10 | 0/10 | — | — | — | — | — | — | — | 12,8 | |
| Daunorubicine<br>libre<br>1 mg/kg<br>J.1.2.3.4. | | | | | | | | 10/10 | 8/10 | 8/10 | 6/10 | 6/10 | 6/10 | 6/10 | 6<br>gué-<br>ries | 19<br>(4 sou-<br>ris) | 40,6 |
| Daunorubicine<br>polymère<br>1 mg/kg<br>J.1.2.3.4. | 9/10 | 7/10 | 6/10 | 4/10 | 4/10 | 4/10 | 4/10 | 3/10 | 3/10 | 2/10 | 2/10 | 2/10 | 2/10 | 1/10 | 1<br>gué-<br>rie | 12,8<br>(9 sou-<br>ris) | 0 |
| Daunorubicine<br>polymère<br>4 mg/kg<br>J.1 | | | | | | | | 10/10 | 8/10 | 8/10 | 8/10 | 7/10 | 6/10 | 6/10 | 6<br>gué-<br>ries | 21<br>(4 sou-<br>ris) | 64 |

* % d'augmentation de survie

Tableau 5

| Jours | Souris survivantes après l'épreuve | | | | | | | | | | | | | MTS | ILS % |
| | 5 | 10 | 11 | 14 | 15 | 16 | 17 | 18 | 19 | 23 | 27 | 32 | 56 | | |
|---|---|---|---|---|---|---|---|---|---|---|---|---|---|---|---|
| Témoins | 8/10 | 7/10 | 1/10 | 1/10 | 0/10 | — | — | — | — | — | — | — | — | 9,1 | — |
| Daunorubicine libre 1 mg/kg J.1.2.3.4. | 10/10 | 10/10 | 10/10 | 9/10 | 8/10 | 6/10 | 4/10 | 3/10 | 3/10 | 3/10 | 3/10 | 3/10 | 30 % guéries | 15,4 | 69,2 |
| Daunorubicine polymère 1 mg/kg J.1.4.7. | 9/10 | 9/10 | 9/10 | 9/10 | 6/10 | 6/10 | 4/10 | 3/10 | 2/10 | 2/10 | 2/10 | 1/10 | 10 % guéries | 12,9 | 41,7 |
| Daunorubicine polymère 0,25 mg/kg J.1.2.3.4. | 10/10 | 10/10 | 10/10 | 10/10 | 6/10 | 5/10 | 4/10 | 4/10 | 3/10 | 2/10 | 2/10 | 2/10 | 20 % guéries | 15,5 | 70,3 |
| Daunorubicine polymère 0,25 mg/kg J.1.3.5.7.9. | 10/10 | 10/10 | 10/10 | 10/10 | 9/10 | 6/10 | 4/10 | 1/10 | 1/10 | 1/10 | 0/10 | — | — | 16,8 | 84,6 |

Tableau 6

| Total mg | | Survivantes après l'épreuve | | | | | | | | | | | | | | | | | | | MTS | ILS % |
|---|---|---|---|---|---|---|---|---|---|---|---|---|---|---|---|---|---|---|---|---|---|---|
| | | J.4 | 5 | 10 | 11 | 12 | 13 | 14 | 15 | 16 | 17 | 18 | 19 | 21 | 22 | 23 | 24 | 25 | 44 | 66 | | |
| 0 | Témoins épreuves | 10/10 | 10/10 | 8/10 | 6/10 | 4/10 | 0/10 | — | — | — | — | — | — | — | — | — | — | — | — | — | 10,8 | — |
| 4 | Témoins daunorubicine libre 1 mg/kg J.1.2.3.4. | 9/10 | 9/10 | 9/10 | 9/10 | 9/10 | 9/10 | 9/10 | 7/10 | 3/10 | 3/10 | 3/10 | 3/10 | 2/10 | 2/10 | 2/10 | 2/10 | 2/10 | 2/10 | 2/10 | 13,2 | 22,2 |
| 2 | Daunorubicine polymère 0,5 mg/kg J.1.2.3.4. | 10/10 | 10/10 | 10/10 | 9/10 | 9/10 | 9/10 | 7/10 | 7/10 | 5/10 | 5/10 | 4/10 | 3/10 | 3/10 | 2/10 | 1/10 | 1/10 | 1/10 | 1/10 | 1/10 | 16 | 48,1 |
| 4 | Daunorubicine polymère 4 mg/kg J.1. | 10/10 | 10/10 | 10/10 | 10/10 | 9/10 | 9/10 | 8/10 | 8/10 | 6/10 | 4/10 | 3/10 | 3/10 | 3/10 | 3/10 | 3/10 | 3/10 | 3/10 | 2/10 | 2/10 | 18,25 | 69 |
| 2 | Daunorubicine greffée 0,5 mg/kg J.1.2.3.4. | 10/10 | 9/10 | 9/10 | | 9/10 | 8/10 | 7/10 | 7/10 | 6/10 | 4/10 | 4/10 | 4/10 | 4/10 | 2/10 | 2/10 | 1/10 | 0/10 | — | — | 17,9 | 65,7 |

0 088 022

## Tableau 7

| | 11 | 12 | 13 | 14 | 15 | 16 | 17 | 18 | 19 | 20 | 21 | 22 | 23 | 27 | MTS | ILS % |
|---|---|---|---|---|---|---|---|---|---|---|---|---|---|---|---|---|
| Témoins épreuve | 4/10 | 1/10 | 0/10 | — | — | — | — | — | — | — | — | — | — | — | 10,5 | |
| Daunorubicine libre 1 mg/kg J.1.2.3.4. | | | 10/10 | 9/10 | 9/10 | 8/10 | 7/10 | 5/10 | 5/10 | 5/10 | 3/10 | 1/10 | 0/10 | — | — 18,2 | 73,3 |
| Daunorubicine polymère 4 mg/kg J.1. | | | | 10/10 | 9/10 | 8/10 | 8/10 | 6/10 | 5/10 | 4/10 | 3/10 | 3/10 | 2/10 | 1/10 | — 20,6 | 96,2 |
| Daunorubicine libre 4 mg/kg - J.1. | | | | | 10/10 | 8/10 | 4/10 | 2/10 | 0/10 | — | — | — | — | — | — 16,4 | 58,2 |

## Tableau 8

| | Nombre de souris survivantes après l'épreuve | | | | | | | | | | | | | | MTS ± SD | ILS % |
|---|---|---|---|---|---|---|---|---|---|---|---|---|---|---|---|---|
| | J9 | 10 | 11 | 12 | 13 | 14 | 15 | 16 | 17 | 18 | 19 | 20 | 21 | 27 | | |
| Essai 1 | 10/10 | 7/10 | 4/10 | 2/10 | 0/10 | — | — | — | — | — | — | — | — | — | 10,5 ± 1,5 | — |
| Essai 2 | | | 10/10 | 9/10 | 9/10 | 9/10 | 8/10 | 6/10 | 2/10 | 0/10 | — | — | — | 16,3 ± 1,8 | 55,2 |
| Essai 3 | | 10/10 | 9/10 | 9/10 | 9/10 | 9/10 | 9/10 | 7/10 | 5/10 | 4/10 | 1/10 | 0/10 | — | 17,2 ± 2,6 | 63,8 |
| Essai 4 | 8/10 | 8/10 | 5/10 | 4/10 | 3/10 | 0/10 | — | — | — | — | — | — | — | — | 10,8 ± 1,9 | 2,8 |
| Essai 5 | | | | | 10/10 | 9/10 | 8/10 | 5/10 | 4/10 | 4/10 | 2/10 | 1/10 | 0/10 | 17,8 ± 3,45 | 69,5 |

**Revendications**

1. Agents antitumoraux à efficacité améliorée, caractérisés en ce qu'ils consistent en le produit de la réaction avec du glutaraldéhyde d'au moins un agent antitumoral, porteur de groupes capables de réagir avec le glutaraldéhyde, en choisissant les conditions de réaction entre l'agent antitumoral à traiter et le glutaraldéhyde, et spécialement la quantité de glutaraldéhyde, en utilisant une quantité minimale de celui-ci, supérieure à celle qui laisse dans le milieu réactionnel des quantités résiduelles non réagies d'agent antitumoral, sans dépasser les quantités excessives de glutaraldéhyde susceptibles de conduire à un produit soluble en milieu aqueux par saturation des groupements réactifs de l'agent antitumoral, aboutissant à un produit insoluble en milieu aqueux, lequel produit, après élimination du glutaraldéhyde éventuel en excès, permet, mis en suspension dans un milieu aqueux, de libérer progressivement l'agent actif sous une forme soluble en conservant son activité antitumorale.

2. Agents antitumoraux selon la revendication 1, caractérisés en ce qu'ils sont formés à partir de la daunorubicine (ou daunomycine) ou de la doxorubicine (ou adriamycine).

3. Procédé pour l'obtention des agents antitumoraux selon l'une des revendications 1 ou 2, dans lequel on soumet l'agent actif à une réaction de polymérisation, caractérisé en ce qu'on met un agent antitumoral porteur de groupes capables de réagir avec le glutaraldéhyde, en contact avec du glutaraldéhyde, en choisissant les conditions de réaction entre l'agent antitumoral à traiter et le glutaraldéhyde, et spécialement la quantité de glutaraldéhyde, en utilisant une quantité minimale de celui-ci, supérieure à celle qui laisse dans le milieu réactionnel des quantités résiduelles non réagies d'agent antitumoral, sans dépasser les quantités excessives de glutaraldéhyde susceptibles de conduire à un produit soluble en milieu aqueux par saturation des groupements réactifs de l'agent antitumoral, et en ce qu'on récupère un produit insoluble en milieu aqueux, lequel produit, après élimination du glutaraldéhyde éventuel en excès, permet, mis en suspension dans un milieu aqueux, de libérer progressivement l'agent actif sous une forme soluble en conservant son activité antitumorale.

4. Procédé selon la revendication 3, caractérisé en ce qu'on met directement en présence, en particulier à température ambiante, l'agent antitumoral à traiter et la quantité convenable de glutaraldéhyde, et en ce qu'on poursuit la réaction jusqu'à la formation d'un produit insoluble.

5. Procédé selon la revendication 3, caractérisé en ce qu'on effectue la réaction de l'agent actif et du glutaraldéhyde en présence d'un support, tel que des cellules, et plus particulièrement des lymphocytes, fournissant un produit insoluble sous forme d'une préparation cellulaire, de préférence lymphocytaire, portant l'agent actif sous forme couplée et pouvant, après mise en suspension dans un milieu aqueux, libérer progressivement l'agent actif sous une forme soluble en conservant son activité antitumorale.

6. Procédé selon l'une quelconque des revendications 3 à 5, caractérisé en ce que, à titre d'agent antitumoral, on utilise la daunorubicine (ou daunomycine) ou la doxorubicine (ou adriamycine).


**Claims**

1. Antitumour agents having improved efficiency characterized in that they consist of the product of reaction of glutaraldehyde with at least one antitumour agent bearing a group capable of reacting with glutaraldehyde, selecting the conditions of reaction between the antitumour agent to be treated and the glutaraldehyde, and especially the amount of glutaraldehyde, using a minimal amount of the latter, this amount being greater than that which leaves unreacted residual amounts of antitumour agent in the reaction medium without exceeding excessive amounts of glutaraldehyde which are likely to lead to a product that is soluble in aqueous medium by saturation of the reactive groups of the antitumour agent. Leading to a product that is insoluble in aqueous medium, which product, after removal of any excess glutaraldehyde, enables, when suspended in an aqueous medium, the active agent to be gradually released in a soluble form while preserving its antitumour activity.

2. Antitumour agents according to Claim 1, characterized in that they are formed from daunorubicin (or daunomycin) or doxorubicin (or adriamycin).

3. Process for producing the antitumour agents according to one of Claims 1 and 2 in which the active agent is subjected to a polymerization reaction, characterized in that an antitumour agent bearing groups capable of reacting with glutaraldehyde is brought into contact with glutaraldehyde, selecting the conditions of reaction between the antitumour agent to be treated and the glutaraldehyde, and especially the amount of glutaraldehyde, using a minimal amount of the latter, this amount being greater than that which leaves unreacted residual amounts of antitumour agent in the reaction medium without exceeding excessive amounts of glutaraldehyde which are likely to lead to a product that is soluble in aqueous medium by saturation of the reactive groups of the antitumour agent, and in that a product that is insoluble in aqueous medium is recovered, which product, after removal of any excess glutaraldehyde, enables, when suspended in an aqueous medium, the active agent to be gradually released in a soluble form while preserving its antitumour activity.

4. Process according to Claim 3, characterized in that the antitumour agent to be treated is brought directly into contact, especially at room temperature, with the appropriate amount of glutaraldehyde, and in that the reaction is continued until an insoluble product is formed.

# 0 088 022

5. Process according to Claim 3, characterized in that the reaction between the active agent and glutaraldehyde is performed in the presence of a support, such as cells, and more especially lymphocytes, yielding an insoluble product in the form of a cell preparation, preferably a lymphocyte preparation, bearing the active agent in coupled form and capable, after suspension in an aqueous medium, of gradually releasing the active agent in a soluble form while preserving its antitumour activity.

6. Process according to any one of Claims 3 to 5, characterized in that, as antitumour agent, daunorubicin (or daunomycin) or doxorubicin (or adriamycin) is used.

**Patentansprüche**

1. Antitumormittel mit verbesserter Wirksamkeit, dadurch gekennzeichnet, daß sie aus dem Produkt der Reaktion wenigstens eines Antitumormittels, das Gruppen enthält, die mit Glutaraldehyd reaktionsfähig sind, mit Glutaraldehyd bestehen, wobei man die Bedingungen der Reaktion zwischen dem zu behandelnden Antitumormittel und Glutaraldehyd, insbesondere die Menge des Glutaraldehyds so wählt, daß man das letztere in minimaler Menge verwendet, die höher ist als die Menge, die im Reaktionsmedium restliche Mengen des Antitumormittels unumgesetzt läßt, ohne übermäßige Mengen des Glutaraldehyds zu überschreiten, die in der Lage sind, zu einem Produkt zu führen, das in wäßrigem Medium durch Sättigung der reaktionsfähigen Gruppen des Antitumormittels löslich ist, wobei man zu einem in wäßrigen Medien unlöslichen Produkt gelangt, das es nach Entfernung des gegebenenfalls überschüssigen Glutaraldehyds ermöglicht, nach Suspendierung in einem wäßrigen Medium progressiv den Wirkstoff in löslicher Form unter Bewahrung seiner Antitumorwirkung freizusetzen.

2. Antitumormittel nach Anspruch 1, dadurch gekennzeichnet, daß sie aus Daunorubicin (oder Daunomycin) oder Doxorubicin (oder Adriamycin) gebildet worden sind.

3. Verfahren zur Herstellung von Antitumormitteln nach einem der Ansprüche 1 oder 2, wobei man den Wirkstoff einer Polymerisationsreaktion unterwirft, dadurch gekennzeichnet, daß man ein Antitumormittel, das Gruppen enthält, die mit Glutaraldehyd reaktionsfähig sind, mit Glutaraldehyd in Berührung bringt, wobei man die Bedingungen der Reaktion zwischen dem zu behandelnden Antitumormittel und dem Glutaraldehyd, insbesondere die Menge des Glutaraldehyds so wählt, daß man das letztere in minimaler Menge verwendet, die höher ist als die Menge, die im Reaktionsmedium restliche Mengen des Antitumormittels unumgesetzt läßt, ohne übermäßige Mengen des Glutaraldehyds zu überschreiten, die in der Lage sind, zu einem Produkt zu führen, das in wäßrigem Medium durch Sättigung der reaktionsfähigen Gruppen des Antitumormittels löslich ist, und daß man ein Produkt gewinnt, das in wäßrigem Medium unlöslich ist und es nach Entfernung des gegebenenfalls überschüssigen Glutaraldehyds ermöglicht, nach Suspendierung in einem wäßrigen Medium progressiv den Wirkstoff in löslicher Form unter Bewahrung seiner Antitumorwirkung freizusetzen.

4. Verfahren nach Anspruch 3, dadurch gekennzeichnet, daß man das zu behandelnde Antitumormittel und die geeignete Menge Glutaraldehyd direkt insbesondere bei Umgebungstemperatur zusammengibt, und daß man die Reaktion bis zur Bildung eines unlöslichen Produkts fortsetzt.

5. Verfahren nach Anspruch 3, dadurch gekennzeichnet, daß man die Reaktion des Wirkstoffs und des Glutaraldehyds in Gegenwart eines Trägers, zum Beispiel Zellen, insbesondere Lymphozyten, durchführt, wobei ein unlösliches Produkt in Form eines zellulären, vorzugsweise lymphocytären Präparats erhalten wird, das den Wirkstoff in gebundener Form enthält und nach Suspendierung in einem wäßrigen Medium den Wirkstoff in löslicher Form unter Bewahrung seiner Antitumorwirkung freizusetzen vermag.

6. Verfahren nach irgendeinem der Ansprüche 3 bis 5, dadurch gekennzeichnet, daß man als Antitumormittel Daunorubicin (oder Daunomycin) oder Doxorubicin (oder Adriamycin) verwendet.

14